# EUROPEAN PATENT SPECIFICATION

(11) **EP 1 560 525 B1**
(45) Date of publication and mention of the grant of the patent: **14.01.2009**
(21) Application number: 03781894.5
(22) Date of filing: 07.11.2003
(51) Int. Cl.: A61B 17/00

(54) **PATENT FORAMEN OVALE (PFO) CLOSURE WITH MAGNETIC FORCE**
VERSCHLUSS EINES PERSISTENTEN SEPTUMSCHADENS MIT MAGNETISCHER KRAFT
FERMETURE DU FORAMEN OVALE PERMEABLE AVEC UNE FORCE MAGNETIQUE

(30) Priority: 07.11.2002 US 424491 P
(43) Date of publication of application: 10.08.2005
(73) Proprietor: NMT Medical, Inc., Boston, MA 02210 (US)
(72) Inventor: OPOLSKI, Steven, W., Carlisle, MA 01741 (US)
(74) Representative: Chapman, Paul William
(86) International application number: PCT/US2003/035998
(87) International publication number: WO 2004/043266

(56) References cited:
- WO-A-03/024337
- US-A- 3 924 631
- US-A- 6 079 414
- US-A1- 2001 037 129
- US-B1- 6 190 353

## Description

This application claims priority from November 7, 2002

### Background of the Invention

A patent foramen ovale (PFO) is a persistent, one-way, typically flap-like opening in a wall between the right atrium and the left atrium of the heart. Left atrial (LA) pressure is typically higher than right atrial (RA) pressure, so the flap typically stays closed. Under certain conditions, however, RA pressure can exceed LA pressure, creating the possibility for right to left shunting that can allow blood clots to enter systemic circulation. In utero, the foramen ovale serves as a physiologic conduit for right-to-left shunting. After birth, with the establishment of pulmonary circulation, the increased left atrial blood flow and pressure results in functional closure of the foramen ovale. This functional closure is subsequently followed by anatomical closure of the two overlapping layers of tissue, referred to as septum primum and septum secundum.

Studies have confirmed a strong association between the presence of a PFO and a risk for paradoxical embolism or stroke. In addition, there is evidence that patients with PFO and paradoxical embolism are at increased risk for future, recurrent cerebrovascular events.

The presence of a PFO has no therapeutic consequence in otherwise healthy adults. In contrast, patients suffering a stroke or TIA in the presence of a PFO and without another cause of ischemic stroke are considered for prophylactic medical therapy to reduce the risk of a recurrent embolic event. These patients are commonly treated with oral anticoagulants, which have the potential for adverse side effects, such as hemorrhaging, hematoma, and interactions with a variety of other drugs. In certain cases, such as when anticoagulation is contraindicated, surgery may be used to close a PFO. To suture a PFO closed requires attachment of septum secundum to septum primum with either a continuous or interrupted stitch, which is a common way a surgeon shuts the PFO under direct visualization.

Nonsurgical closure of PFOs has become possible with the advent of umbrella devices and a variety of other similar mechanical closure designs, developed initially for percutaneous closure of atrial septal defects (ASD). These devices allow patients to avoid the potential side effects often associated with anticoagulation therapies.

US-A-3 924 631 discloses a device with two magnets attracting each other and being encased in a hinged pair of jaws. Temporary compression of the urethra is suggested in column 1 but due to the hinge the entire periphery of a hole cannot be closed off.

US-B-6 190 353 figure 9-d discloses a device where connectors between two magnets are used to maintain an opening between two blood-vessels.

### Summary of the Invention

The present invention includes the use of magnetic force, preferably with one or more permanent (non-electromagnetic) magnets, to hold together flaps of tissue inside the body, particularly a PFO. Magnets, such as rare earth magnets, that develop high attractive forces when separated with a material or air gap are preferably used. It can be preferable for a number of magnets to be provided in a conduit to provide some flexibility. If desired, after a period of time, such as a few weeks, the entire device or the magnets within the device can be retrieved such that no permanent implant is left behind.

The invention may be used in methods for using magnetic force, including deploying a magnet on one side of a region to be treated, deploying a magnetically attractive piece that is attractive to the magnet (and which might or might not be a magnet), with the magnet and magnetically attractive piece part of a device such as a septal occluder or a PFO closure device.

The use of magnets with a sheath or container adapted for in-growth can promote healing and potentially allow the PFO to close, preferably with a very small device in terms of diameter and metal mass. Other features will become apparent from the drawings, description, and claims.

### Brief Description of Drawings

Fig. 1 is a cross-sectional view of a conduit with magnets.
Fig. 2 is a side view of components on either side of a PFO where one or both components include magnets.
Fig. 3 is a top view of components on either side of a PFO where one or both components include magnets.
Fig. 4 shows a device with magnets at the end of petals, in a catheter for deployment.
Fig. 5 shows a device with petals as deployed.
Fig. 6 shows a device similar to that in Fig. 4, in a catheter for deployment.

### Detailed Description

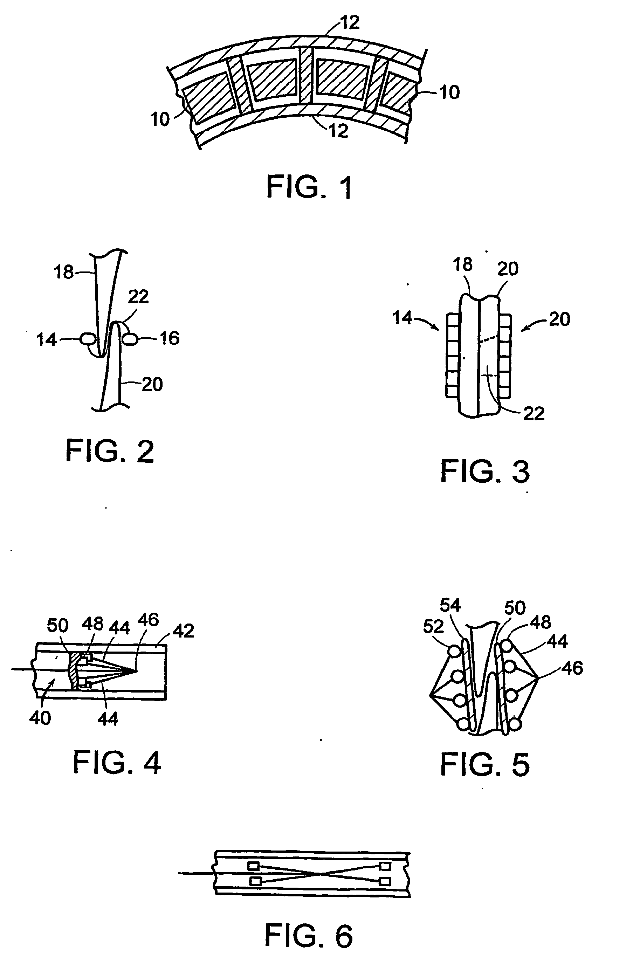

Referring to Fig. 1, one or more magnets can be used to provide magnetic force with sufficient attractive force to hold together the flaps of a PFO, and preferably to cause regrowth between the flaps, but not too much to create tissue damage. To provide a locally strong magnetic field without bulky weight, it is desirable to use small, rare earth magnets, although other magnets could be used. While magnets are typically rigid, some flexibility can be provided in the magnetic structure by using a group of magnets, such as a short length of magnets 10, in a flexible conduit 12. The magnets can be connected together, such as with a wire, or can be separated by walls within the conduit, or can even be loose within a conduit. A conduit for holding magnets, or selected portions thereof, can be made of bioresorbable material, or can be made with materials, sizes, and/or coatings that promote or hinder in-growth, depending on the way in which it is being used. Examples of materials that can be used and that promote in-growth include vascular graft material, such as knitted or woven polyester, expanded PTFE, polyurethane, or polyvinyl alcohol (PVA).

Referring also to Fig. 2, two magnets, or preferably sets of magnets 14, 16, with each set in a conduit such as that shown generally in Fig. 1, can be provided on either side of the PFO as defined by flaps 18, 20 (septum secundum and septum primum) with a connector 22, such as a wire or a polymer fabric scaffold. The magnetically attractive force forms a line of contact along the flap of the PFO. Alternatively, a magnet can be used on one side of the PFO with only a magnetically attractive material, such as a metal, on the other side of the PFO. The conduit and magnets are typically inserted through the use of a catheter.

The magnets can be left in permanently, in which case it would be desirable to promote in-growth around the conduit. One drawback to the use of magnets in the body on a permanent basis, however, is that their presence would limit the use of MRI (magnetic resonance imaging).

By making the magnets retrievable, MRI could be used later for a patient that had magnets removed. A conduit (such as that shown in Fig. 1) into which magnets are placed is designed to limit or restrict the amount of tissue in-growth to the tube. This limitation of in-growth can be effected by the selection of mesh size, choice of materials, or use of a coating on the conduit. The magnets can alternatively be provided in an inner sheath within an outer sheath that is made of a material and/or with a design to encourage tissue in-growth into and around the sheath. This means that the tissue can grow together around the sheath. A subsequent procedure is used to pull the magnets and inner sheath from the outer sheath by either sliding the inner sheath out from the outer sheath. Alternatively, the outer sheath can be made bioresorbable, and the inner sheath is removed before it has been resorbed.

Referring to Fig. 4, a portion of a device 40 is shown in a catheter 42. The device has a number of wires 44 (shown here as four in number), connected at a hub 46. At the end of wires 44 are magnets 48, and against the magnets is a fabric 50. The magnets can be oriented to have a repulsive force. Referring also to Fig. 5, as deployed, the repulsive force of the magnets causes the wires connected to the fabric 50 to spread out against one side of the PFO. A second device can be provided each with magnets 52 against a fabric 54, with magnets 52 having an orientation that causes an attractive force to magnets 48. As a result, magnets 52 and 48 are attracted to each other to help hold the PFO closed. The wires can be made of a shape memory material, such as nitinol.

Fig. 6 shows a device similar to that shown in Fig. 4 in which the device is half folded on itself to reduce the profile of the device within the catheter. A fabric can be used in this case if desired.

Other methods can be used whereby petals or other structures are created, taking advantage of the attractive and repulsive forces of magnets.

The strength of the magnets and the size and shape of the magnets and conduit can be determined experimentally, taking into consideration the gap between the materials on either side of the PFO.

Accordingly, the present invention has been described with respect to exemplary embodiments of the present invention. It should be appreciated, though, that the present invention is defined by the following claims. Modifications or changes may be made to the exemplary embodiments of the present invention without departing from the scope of the claims.

## Claims

1. A patent foramen ovale (PFO) closure device comprising a first magnet (14) and a block, the block and the first magnet (14) having an attractive force, the device being deployable in a living body such that the first magnet (14) and the block are on opposite sides of the PFO, and use magnetic force to hold together septum primum (20) and septum secundum (18) of the PFO.

2. The device of claim 1, wherein the block includes a second magnet (16).

3. The device of claim 2, wherein the first and second magnets (14 and 16 respectively) are held in respective first and second sheaths.

4. The device of claim 3, wherein the first and second sheaths can be coupled together.

5. The device of claim 4, wherein the sheaths are coupled with a connector (22) adapted to extend from one side of a PFO to another between septum primum (20) and septum secundum (18).

6. The device of any of claims 3 to 5, wherein the first and second sheaths are within an outer sheath.

7. The device of claim 6, wherein the outer sheath is adapted to encourage tissue in-growth.

8. The device of claim 6, wherein the outer sheath is made of a bioresorbable material.

9. The device of any of claims 2 to 8, wherein the device includes a plurality of magnets (10) in a conduit (12) to form a flexible magnetic structure.

10. The device of any of claims 1 to 9, further comprising a catheter (42) for delivering the closure device, the closure device being movable from a delivery position in which the device can be provided within the catheter to a deployed position in the living body.

11. The device of any of claims 1 to 10, wherein the device has an outer container and an inner container for holding one or more magnets, the inner container being removable from the outer container.

12. The device of claim 11, wherein the outer sheath is adapted to encourage in-growth, and the inner container is adapted to inhibit in-growth.

13. The device of claim 12, wherein the inner and outer sheaths are adapted to inhibit and encourage in-growth by the use of different materials.

## Patentansprüche

1. Verschlussvorrichtung für ein persistierendes Foramen ovale (PFO) mit einem ersten Magneten (14) und einem Block, wobei der Block und der erste Magnet (14) eine Anziehungskraft aufweisen, die Vorrichtung in einen lebenden Körper einsetzbar ist, so dass der erste Magnet (14) und der Block an gegenüberliegenden Seiten des PFO angeordnet sind und Magnetkraft verwenden, um das Septum primum (20) und das Septum secundum (18) des PFO zusammenzuhalten.

2. Vorrichtung nach Anspruch 1, wobei der Block einen zweiten Magneten (16) aufweist.

3. Vorrichtung nach Anspruch 2, wobei die ersten und zweiten Magneten (14 bzw. 16) in jeweiligen ersten und zweiten Hüllen gehalten werden.

4. Vorrichtung nach Anspruch 3, wobei die ersten und zweiten Hüllen miteinander verbunden sein können.

5. Vorrichtung nach Anspruch 4, wobei die Hüllen mit einem Verbindungsmittel (22) verbunden sind, das eingerichtet ist, um sich von einer Seite eines PFO zur anderen zwischen dem Septum primum (20) und dem Septum secundum (18) zu erstrecken.

6. Vorrichtung nach einem der Ansprüche 3 bis 5, wobei die ersten und zweiten Hüllen innerhalb einer äußeren Hülle liegen.

7. Vorrichtung nach Anspruch 6, wobei die äußere Hülle eingerichtet ist, um das Einwachsen von Gewebe zu fördern.

8. Vorrichtung nach Anspruch 6, wobei die äußere Hülle aus einem bioresorbierbaren Material hergestellt ist.

9. Vorrichtung nach einem der Ansprüche 2 bis 8, wobei die Vorrichtung in einem Kanal (12) eine Mehrzahl von Magneten (10) enthält, um eine flexible Magnetstruktur zu bilden.

10. Vorrichtung nach einem der Ansprüche 1 bis 9, weiters mit einem Katheter (42) zum Zuführen der Verschlussvorrichtung, wobei die Verschlussvorrichtung von einer Zuführposition, in welcher die Vorrichtung innerhalb des Katheters bereitgestellt werden kann, in eine im lebenden Körper eingesetzte Position bewegbar ist.

11. Vorrichtung nach einem der Ansprüche 1 bis 10, wobei die Vorrichtung ein äußeres Behältnis und ein inneres Behältnis zum Halten eines oder mehrerer Magneten aufweist, wobei das innere Behältnis aus dem äußeren Behältnis entfernbar ist.

12. Vorrichtung nach Anspruch 11, wobei die äußere Hülle eingerichtet ist, um das Einwachsen zu fördern, und das innere Behältnis eingerichtet ist, um das Einwachsen zu hemmen.

13. Vorrichtung nach Anspruch 12, wobei die inneren und äußeren Hüllen eingerichtet sind, um das Einwachsen durch Verwenden von unterschiedlichen Materialien zu hemmen und zu fördern.

## Revendications

1. Dispositif de fermeture du foramen ovale perméable (PFO) comprenant un premier aimant (14) et un bloc, le bloc et le premier aimant (14) ayant une force d'attraction, le dispositif étant déployable dans un corps vivant de telle sorte que le premier aimant (14) et le bloc soient sur des côtés opposés du PFO, et l'utilisation d'une force magnétique pour maintenir conjointement le septum primum (20) et le septum secundum (18) du PFO.

2. Dispositif selon la revendication 1, dans lequel le bloc comprend un deuxième aimant (16).

3. Dispositif selon la revendication 2, dans lequel les premier et deuxième aimants (14 et 16, respectivement) sont maintenus dans des première et deuxième gaines respectives.

4. Dispositif selon la revendication 3, dans lequel les première et deuxième gaines peuvent être couplées l'une à l'autre.

5. Dispositif selon la revendication 4, dans lequel les gaines sont couplées à un connecteur (22) adapté pour s'étendre d'un côté d'un PFO à un autre entre le septum primum (20) et le septum secundum (18).

6. Dispositif selon l'une quelconque des revendications 3 à 5, dans lequel les première et deuxième gaines sont à l'intérieur d'une gaine externe.

7. Dispositif selon la revendication 6, dans lequel la gaine externe est adaptée pour favoriser la croissance tissulaire.

8. Dispositif selon la revendication 6, dans lequel la gaine externe est constituée d'un matériau biorésorbable.

9. Dispositif selon l'une quelconque des revendications 2 à 8, le dispositif comprenant une pluralité d'aimants (10) dans un conduit (12) pour former une structure magnétique flexible.

10. Dispositif selon l'une quelconque des revendications 1 à 9, comprenant en outre un cathéter (42) pour distribuer le dispositif de fermeture, le dispositif de fermeture étant mobile à partir d'une position de distribution dans laquelle le dispositif peut être fourni dans le cathéter jusqu'à une position déployée dans le corps vivant.

11. Dispositif selon l'une quelconque des revendications 1 à 10, le dispositif ayant un conteneur externe et un conteneur interne pour maintenir un ou plusieurs aimants, le conteneur interne pouvant être retiré du conteneur externe.

12. Dispositif selon la revendication 11, dans lequel la gaine externe est adaptée pour favoriser la croissance et le conteneur interne est adapté pour inhiber la croissance.

13. Dispositif selon la revendication 12, dans lequel les gaines interne et externe sont adaptées pour inhiber et favoriser la croissance par l'utilisation de différents matériaux.
